# EUROPEAN PATENT SPECIFICATION

(11) **EP 0 762 875 B1**
(45) Date of publication and mention of the grant of the patent: **20.12.2000**
(21) Application number: 95918482.1
(22) Date of filing: 18.05.1995
(51) Int. Cl.: A61K 9/58

(54) **MICROPARTICLE DELIVERY SYSTEM**
MIKROPARTIKEL MIT ABGABESYSTEM
SYSTEME DE LIBERATION DE MICROPARTICULES

(30) Priority: 18.05.1994 US 245646
(43) Date of publication of application: 19.03.1997
(73) Proprietor: McMASTER UNIVERSITY, Hamilton Ontario L8N 3Z5 (CA)
(72) Inventor: McDERMOTT, Mark, R., Ancaster, Ontario L9G 4T4 (CA); BROOK, Michael, A., Ancaster, Ontario L9G 4M4 (CA); HERITAGE, Philippa, L., Dundas, Ontario L9H 1C7 (CA); UNDERDOWN, Brian, J., Dundas, Ontario L9H 2W1 (CA); LOOMES, Lesley, M., West Ferry, Dundee, DD5 1PW (GB); JIANG, Jianxiong, Hamilton, Ontario L8S 3M7 (CA)
(74) Representative: Smart, Peter John
(86) International application number: PCT/CA95/00294
(87) International publication number: WO 95/31187

(56) References cited:
- EP-A- 0 256 933
- EP-A- 0 271 979
- US-A- 5 075 109

## Description

The present invention relates to a particulate carrier for delivering materials having biological activity. The term "microparticle" as used herein refers to any particulate carrier used for delivery of a biologically-active material and includes materials which are microcapsules and microspheres.

### REFERENCE TO RELATED APPLICATION

This application is a continuation-in-part of copending U.S. patent application serial no. 08/245,646, filed May 18, 1994.

### BACKGROUND OF THE INVENTION

Vaccines have been used for many years to protect humans and animals against a wide variety of infectious diseases. Such conventional vaccines consist of attenuated pathogens (for example, polio virus), killed pathogens (for example, Bordetella pertussis) or immunogenic components of the pathogen (for example, diphtheria toxoid). Some antigens are highly immunogenic and are capable alone of eliciting protective immune responses. Other antigens, however, fail to induce a protective immune response or induce only a weak immune response. This low immunogenicity can be significantly improved if the antigens are co-administered with adjuvants. Adjuvants enhance the immunogenicity of an antigen but are not necessarily immunogenic themselves. Adjuvants may act by retaining the antigen locally near the site of administration to produce a depot effect facilitating a slow, sustained release of antigen to cells of the immune system. Adjuvants can also attract cells of the immune system to an antigen depot and stimulate such cells to elicit immune responses. Adjuvants have been identified that enhance the immune response to antigens delivered parenterally. Some of these adjuvants are toxic, however, and can cause undesirable side-effects, making them unsuitable for use in humans and many animals. Indeed, only aluminum hydroxide and aluminum phosphate are routinely used as adjuvants in human and veterinary vaccines. However, even these adjuvants are not suitable for use with all antigens and can also cause irritation at the site of injection. There is a clear need to develop novel adjuvants which are sate and efficacious for enhancing the immunogenicity of antigens.

Immunization can also be achieved by the delivery of antigens to mucosal surfaces, such as by ingestion of the antigen. Thus, it is known that the ingestion of antigens by animals can result in the appearance of antigen-specific secretory IgA antibodies in intestinal, bronchial or nasal washings and other external secretions. For example, studies with human volunteers have shown that oral administration of influenza vaccine is effective at inducing secretory anti-influenza antibodies in nasal secretions and substances have been identified which might be useful as adjuvants for such ingested vaccines. However, most of these adjuvants are relatively poor in terms of improving immune responses to ingested antigens. Currently, some of these adjuvants have been determined to be safe and efficacious in enhancing immune responses in humans and animals to antigens that are administered via the orogastrointestinal, nasopharyngeal-respiratory and genital tracts or in the ocular orbits. However, administration of antigens via these routes is generally ineffective in eliciting art immune response. The inability to immunize at the mucosal surface is generally believed to be due to:
the destruction of the antigen or a reduction in its immunogenicity in the acidic and/or enzymatically hostile environments created by secretions produced at the mucosal epithelium;
the dilution of the antigen to a concentration that is below that required to induce immune responses; the carriage of antigen from the body in discharges originating at the mucosal epithelium; and
the lack of suitable adjuvants which remain active at the mucosal epithelium.

Clearly, there is a need to identify powerful adjuvants which are safe and efficacious for use at the mucosal epithelium in the orogastrointestinal, nasopharyngeal-respiratory and urogenital tracts and in the ocular orbits and at other mucosal sites.

Sensitive antigens may be entrapped to protect them against destruction, reduction in immunogenicity or dilution. The antigen can be coated with a single wall of polymeric material or can be dispersed within a monolithic matrix. Thus, U.S. Patent 5,151,264 describes a particulate carrier of a phospholipid/glycolipid/polysaccharide nature that has been termed Bio Vecteurs Supra Moléculaires (BVSM). The particulate carriers are intended to transport a variety of molecules having biological activity in one of the layers thereof. However, U.S. Patent 5,151,264 does not describe particulate carriers containing antigens for immunization and particularly does not describe particulate carriers for immunization via the orogastrointestinal, nasopharyngeal-respiratory and urogenital tracts and in the ocular orbits or other mucosal sites.

U.S. Patent 5,075,109 describes encapsulation of the antigens trinitrophenylated keyhole limpet hemocyanin and staphylococcal enterotoxin B in 50:50 poly (DL-lactide-co-glycolide). Other polymers for encapsulation are suggested, such as poly(glycolide), poly(DL-lactide-co-glycolide), copolyoxalates, polycaprolactone, poly(lactide-co-caprolactone), poly(esteramides), polyorthoesters and poly(8-hydroxybutyric acid), and polyanhydrides. The encapsulated antigen was administered to mice via gastric intubation and resulted in the appearance of significant antigen-specific IgA antibodies in saliva and gut secretions and in sera. As stated in this patent, in contrast, the oral administration of the same amount of unencapsulated antigen was ineffective at inducing specific antibodies of any isotype in any of the fluids tested. Poly(DL-lactide-co-glycolide) microcapsules were also used to administer antigen by parenteral injection.

Published PCT application WO 91/06282 describes a delivery vehicle comprising a plurality of bioadhesive microspheres and antigenic vaccine ingredients. The microspheres being of starch, gelatin, dextran, collagen or albumin. This delivery vehicle is particularly intended for the uptake of vaccine across the nasal mucosa. The delivery vehicle may additionally contain an absorption enhancer. The antigens are typically encapsulated within protective polymeric materials.

### SUMMARY OF THE INVENTION

The present invention is directed towards the provision of a new and useful microparticle delivery system, which may be used for delivery of materials having biological activity, including antigens to a host.

In accordance with one aspect of the present invention, there is provided a particulate carrier, which comprises:
a solid core comprising a polysaccharide and a proteinaceous material; and
an organometallic polymer bonded to the core. Such particulate carrier generally has a particle size from about 10 nm to about 50 µm, preferably from about 1 to about 10 µm.

The polysaccharide component of the core may be dextran, starch, cellulose or derivatives thereof, particularly soluble starch. The starch may be derived from a variety of monocotyledonous and dicotyledonous species, such as corn, potato or tapioca.

The proteinaceous material component of the core may have biological activity. An additional material having biological activity also may be included in the core. The particles then provide a delivery vehicle for the biologically-active material to a host, generally an animal, including a human.

The material having biological activity, for example, immunogenicity, includes proteins (such as influenza viral protein), peptides, antigens, bacteria, bacterial lysates, viruses (such as, influenza virus), virus-infected cell lysates (such as, a herpes simplex virus-infected cell lysate), antibodies, carbohydrates, nucleic acids, lipids, haptens, pharmacologically-active materials, and combinations, derivations and mixtures thereof.

The organometallic polymer bonded to the core preferably is derived from a functionalized silicone, including an end-substituted silicone. One particular class of end-substituted silicones from which the organometallic polymer may be derived are (trialkoxysilyl) alkyl-terminated polydialkylsiloxanes.

In a further aspect of the present invention, there is provided an immunogenic composition formulated for mucosal or parenteral administration, comprising the particulate carrier containing an immunogenic material and a physiologically-acceptable carrier therefor.

In an additional aspect, there is provided a method of producing an immune response in a host, comprising the administration thereto, generally by mucosal or parenteral administration, the immunogenic composition provided herein. The immune response produced may be an antibody response, including local and serum antibody responses.

In a further aspect of the present invention, there is provided a method for producing a particulate carrier, which comprises:
(a) forming an aqueous composition comprising a dissolved polysaccharide and a dispersed or dissolved proteinaceous material;
(b) forming an emulsion in which the aqueous composition is the dispersed phase;
(c) forming from the emulsion a particulate carrier comprising a core of said polysaccharide and proteinaceous material having bonded thereto an organometallic polymer; and
(d) collecting the particulate carrier so formed.

The method may optionally include a step of sonicating the suspension of microspheres to produce a fine suspension before the forming step (c), so as to control particle size.

This procedure enables the proteinaceous material to be incorporated into the microparticles under temperature conditions which do not denature the proteinaceous material or adversely affect the biological activity thereof.

Advantages of the present invention include:
(a) ease and safety of microparticle manufacture;
(b) biocompatability and safety of the microparticles;
(C) improved immunogenicity of antigens presented to cells of the immune system by the microparticles;
(d) ease of storage and administration; and
(e) fabrication conditions that do not adversely affect the biological activity of proteinaceous or other material.

In this application, the term "coated" microparticles is used to define microparticles that have a long chain organometallic polymer bound, bonded or otherwise associated with the core thereof.

### BRIEF DESCRIPTION OF THE DRAWINGS

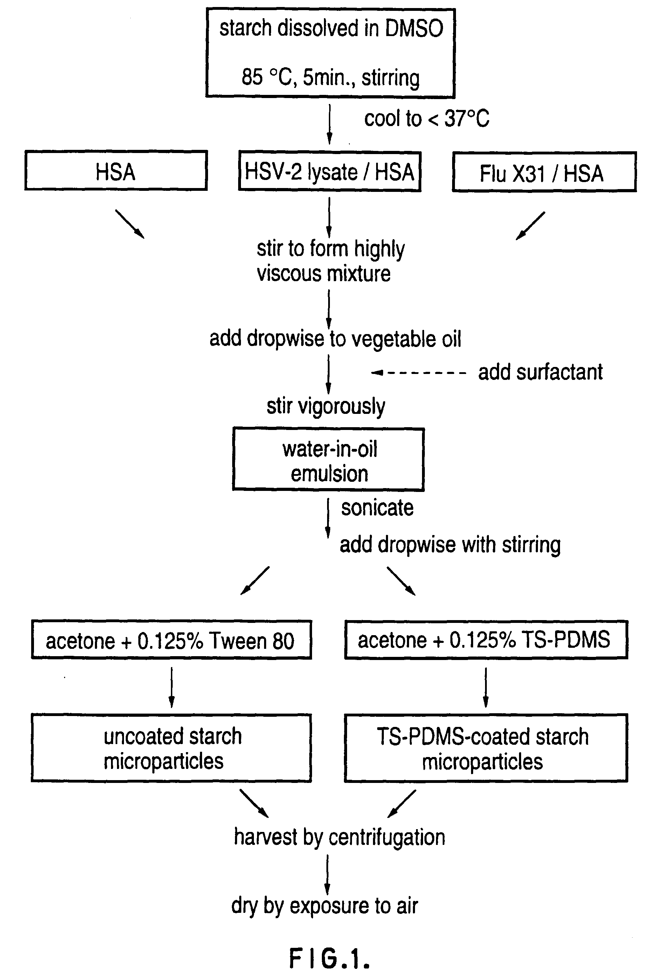

Figure 1 shows a flow diagram for a process for the production of starch microparticles according to one embodiment of the invention. In this Figure, HSA = human serum albumin, HSV2-lysate/HSA - herpes simplex virus type-2 lysate mixed with human serum albumin, Flu X31/HSA = influenza virus strain X31 mixed with human serum albumin.

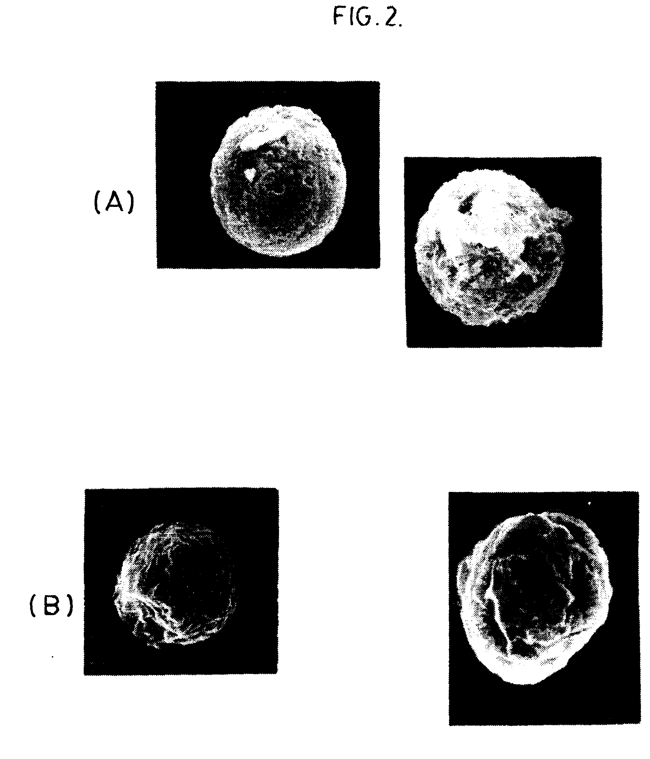

Figure 2 shows scanning electron microscopy (SEM) analysis of influenza virus strain A-X31 and human serum albumin - containing microparticles that were either (A) coated with the silicone polymer s(triethoxysilyl)propyl-terminated polydimethysiloxane (TS-PDMS) or (B) were uncoated. The SEM images represent magnification of 2500 diameters. The nominal diameter of the TD-PDMS-coated microparticles was 10 µm and that of uncoated microparticles was 10 µm.

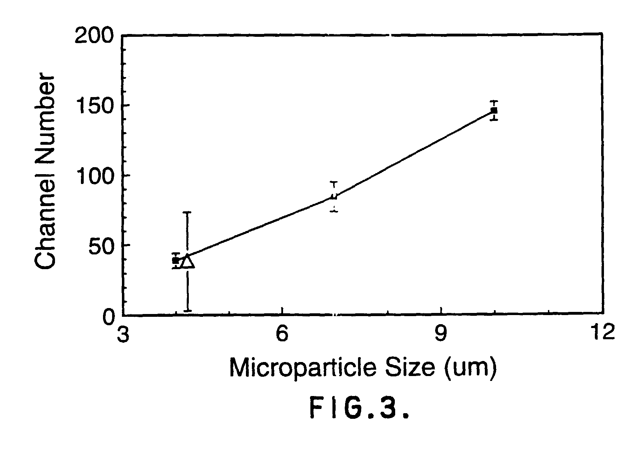

Figure 3 shows the diameter distribution of human serum albumin-containing starch microparticles coated with the silicone polymer 3-(triethoxyl) silylpropyl-terminated polydimethylsiloxane (TS-PDMS). HSA-containing starch particles (Δ) were fabricated and compared to polystyrene microsphere standards by flow cytometry ( -■-; 10 µm, 7 µm, 4 µm diameter). The particles had a mean diameter of 4.18 µm and a standard deviation of plus or minus 3 µm.

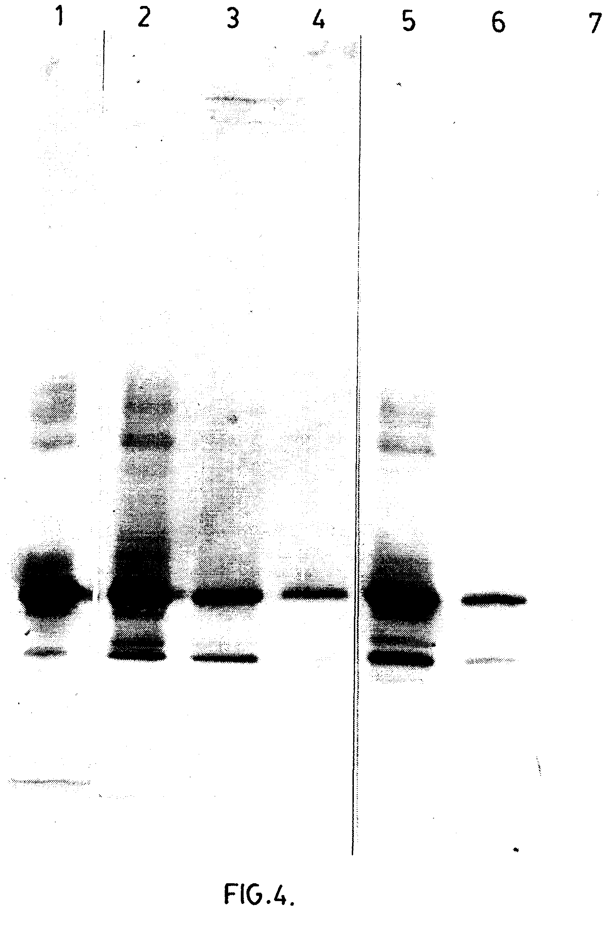

Figure 4 shows an immunoblot analysis of human serum albumin released from human serum albumin-containing starch microparticles that were either coated with the silicone polymer 3-(triethoxysilyl)propyl-terminated polymethylsiloxane (TS-PDMS) or uncoated following suspension of the microparticles in phosphate buffered saline (PBS). Lane 1 shows 0.5 µg of an HSA standard. Lanes 2 to 4 show HSA released from TS-PDMS coated microparticles incubated in vitro for 30 min, 1h and 3h in PBS in vitro and lanes 5 to 7 show HSA released from uncoated microparticles at 30 min., 1h and 3h in vitro.

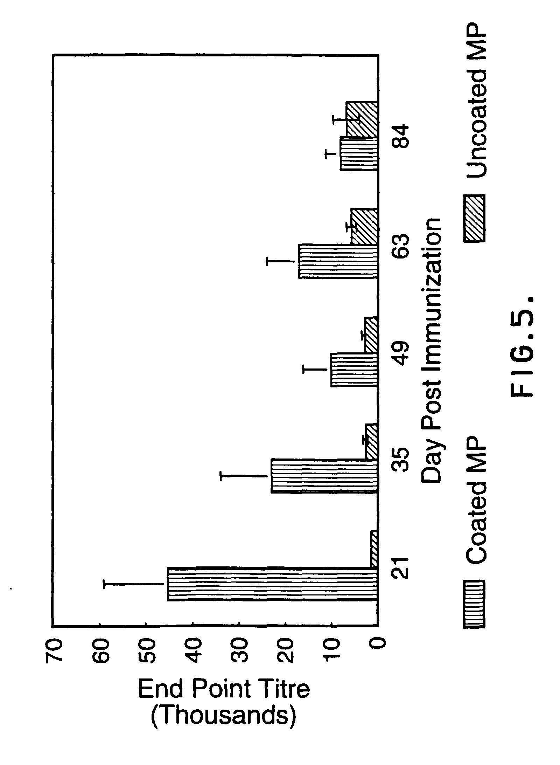

Figure 5 shows the anti-HSA IgG serum antibody responses following various immunization protocols. Groups of 6 mice were immunized intraperitoneally (I.P.) on days 0, 7 and 14 with 250 µL of PBS, pH 7.4, containing 100 µg of HSA incorporated into TS-PDMS-coated or uncoated starch microparticles. Sera obtained on days 21, 35, 49, 63 and 84 were evaluated for the presence of anti-HSA IgG antibodies using an enzyme-linked immunosorbent assay (ELISA). 1 mg of coated or uncoated microparticles contains 50 µg of HSA.

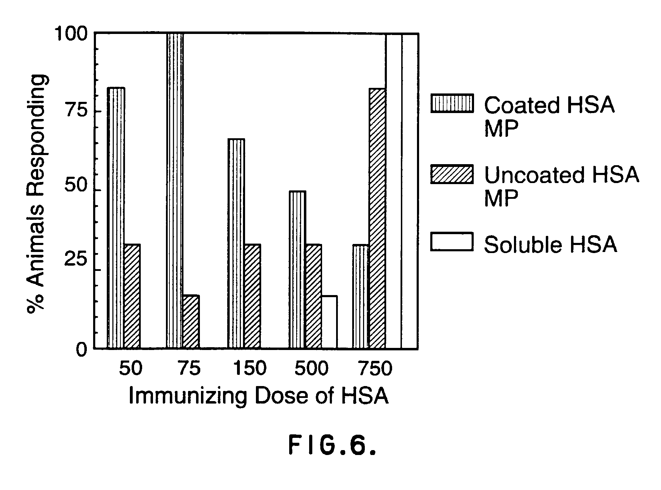

Figure 6 shows the percentage of animals developing an anti-HSA IgG serum antibody response following intragastric immunization with HSA incorporated into uncoated or TS-PDMS coated microparticles as compared to soluble HSA in free form.

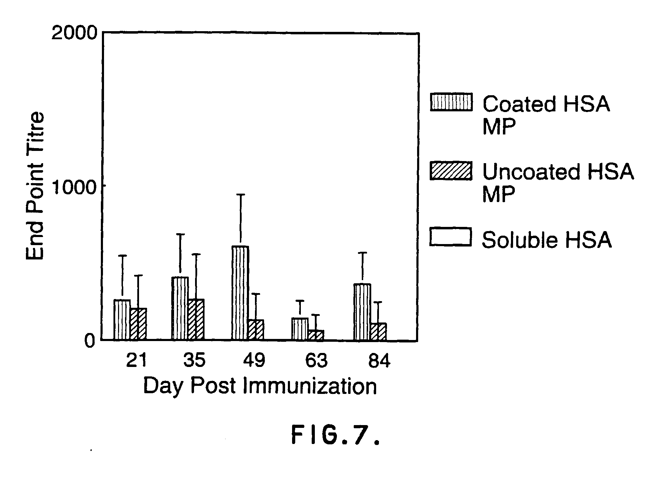

Figure 7 shows the anti-HSA IgG serum antibody titres in six mice immunized intragastrically with a 50 µg dose of uncoated or TS-PDMS coated microparticles. Animals were immunized on days 0, 7 and 14 with 0.5 mL of 0.2 M NaHCO₃ containing 50 µg of HSA incorporated into TS-PDMS-coated or uncoated starch microparticles or soluble HSA. Sera obtained on days 21, 35, 49, 63 and 84 were evaluated for the presence of anti-HSA IgG antibodies using an ELISA. 1 mg of coated or uncoated microparticles contains 50 µg of HSA.

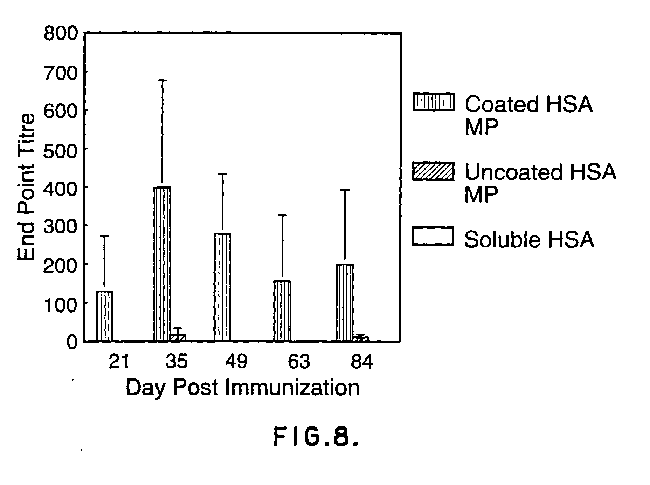

Figure 8 shows the anti-HSA IgG serum antibody titres in six mice immunized intragastrically with a 75 µg dose of uncoated or TS-PDMS coated microparticles as compared to soluble HSA in free form. Animals were immunized on days 0, 7 and 14 with 0.5 mL of 0.2 M NaHCO₃ containing 75 µg of HSA incorporated into TS-PDMS-coated or uncoated starch microparticles or soluble HSA. Sera obtained on days 21, 35, 49, 63 and 84 were evaluated for the presence of anti-HSA IgG antibodies using an ELISA. 1 mg of coated or uncoated microparticles contains 50 µg of HSA.

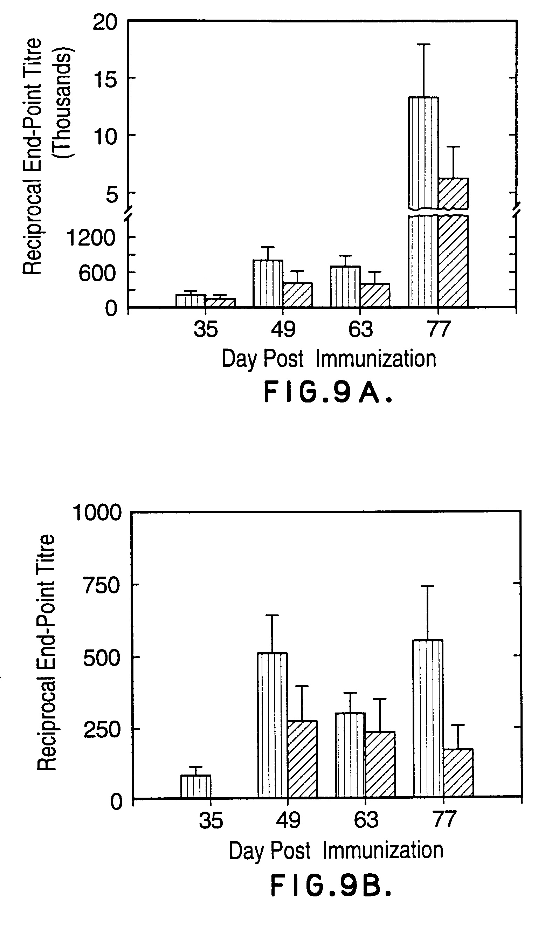

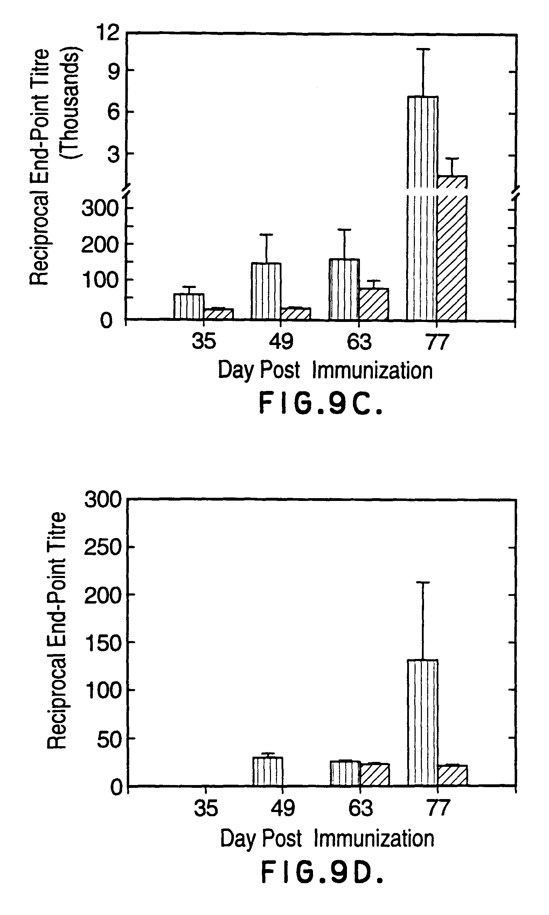

Figure 9 shows the anti-HSA IgG and IgA serum antibody titre in groups of 15 mice immunized intragastrically with 50 µg (Panels A and B) or 10 µg (Panels C and D) of HSA containing microparticles. Animals were immunized on days 0, 7, 14 and 70 with HSA incorporated into TS-PDMS-grafted (solid bars) or ungrafted (hatched bars) microparticles. Sera obtained on days 35, 49, 63 and 77 were evaluated for the presence anti-HSA IgG (Panels A and C) or IgA (Panels B and D) using an ELISA.

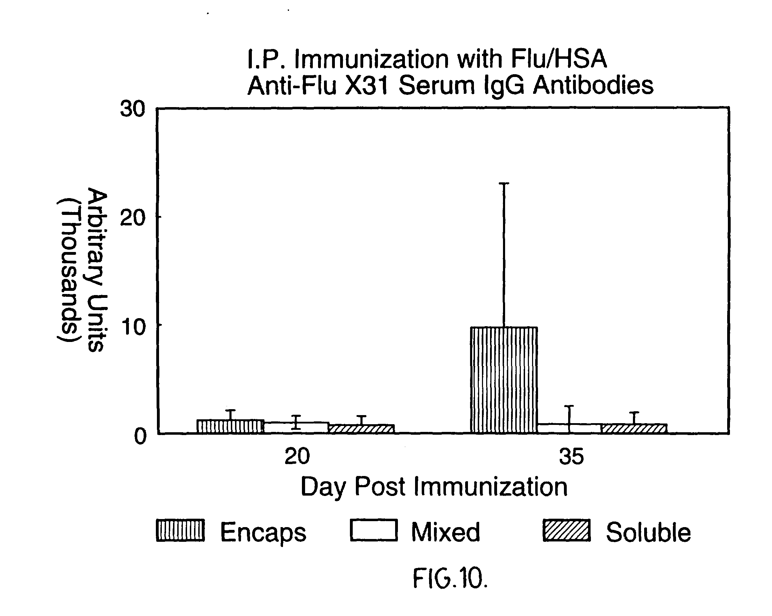

Figure 10 shows the anti-Flu X31 (i.e. influenza virus type A strain X31) serum antibody titres in mice immunized by the intraperitoneal route with soluble Flu X31/HSA, Flu X31/HSA mixed with microparticles coated with TS-PDMS or Flu X31/HSA entrapped in TS-PDMS-coated microparticles.

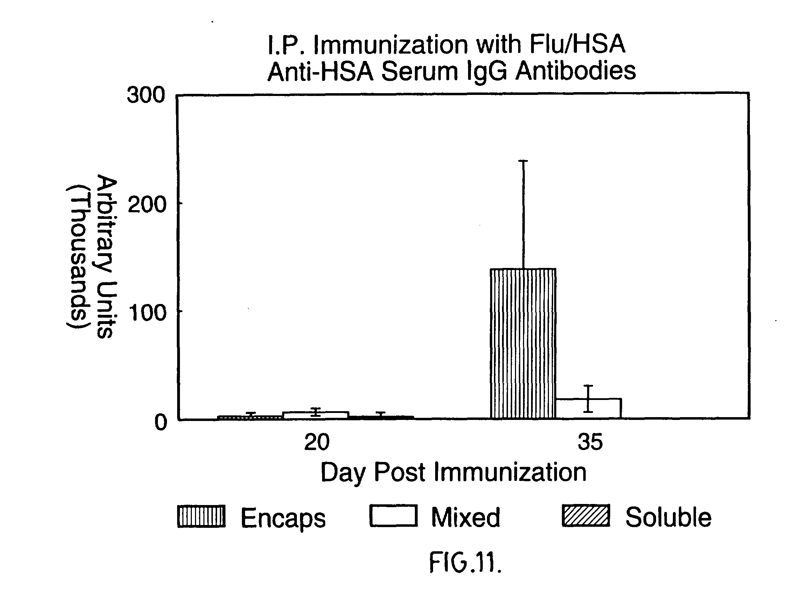

Figure 11 shows the anti-HSA antibody titres in the sera of mice immunized by the intraperitoneal route with soluble Flu X31/HSA, Flu X31/HSA in buffer or mixed with microparticles coated with TS-PDMS or Flu X31/HSA entrapped in TS-PDMS-coated microparticles.

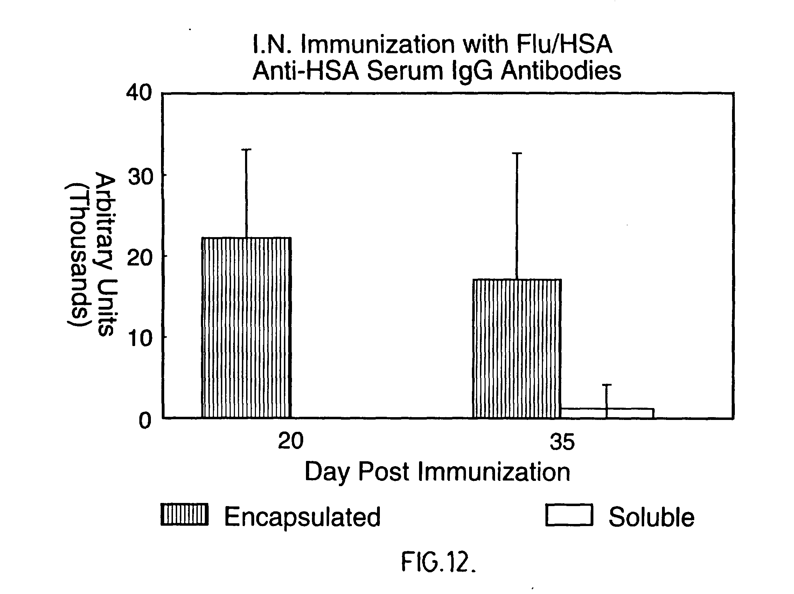

Figure 12 shows the anti-Flu X31 antibody titres in the sera of mice immunized by the intranasal route with soluble Flu X31/HSA or Flu X31/HSA entrapped in TS-PDMS-coated microparticles.

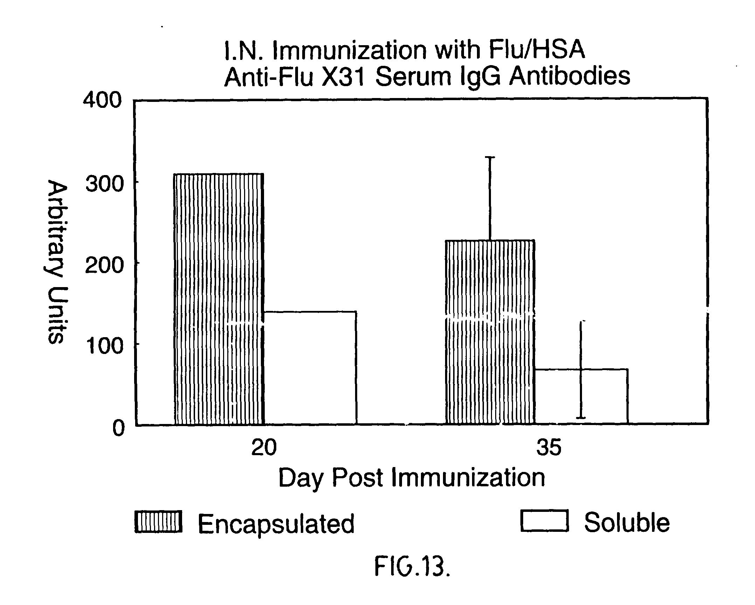

Figure 13 shows the anti-HSA antibody titres in the sera of mice immunized by the intranasal route with soluble Flu X31/HSA or Flu X31/HSA entrapped in TS-PDMS-coated microparticles.

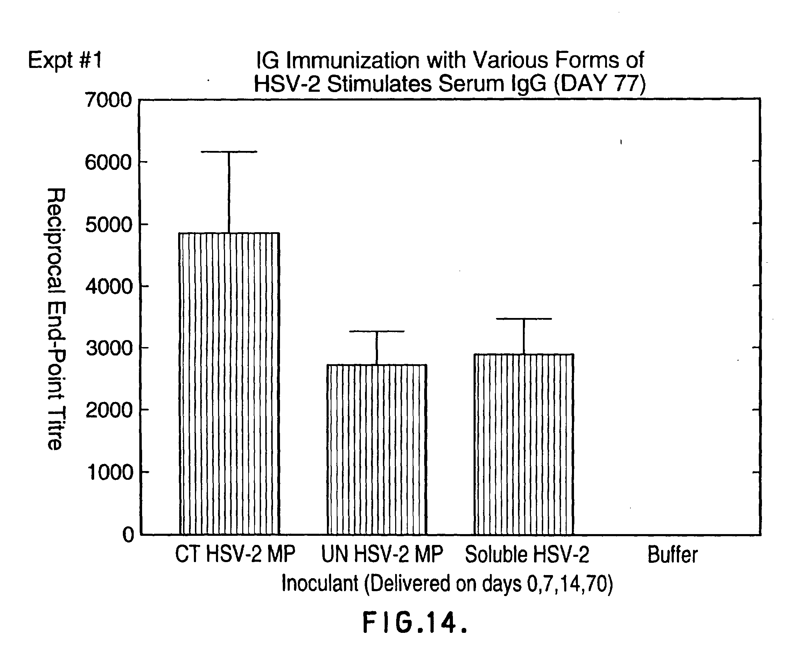

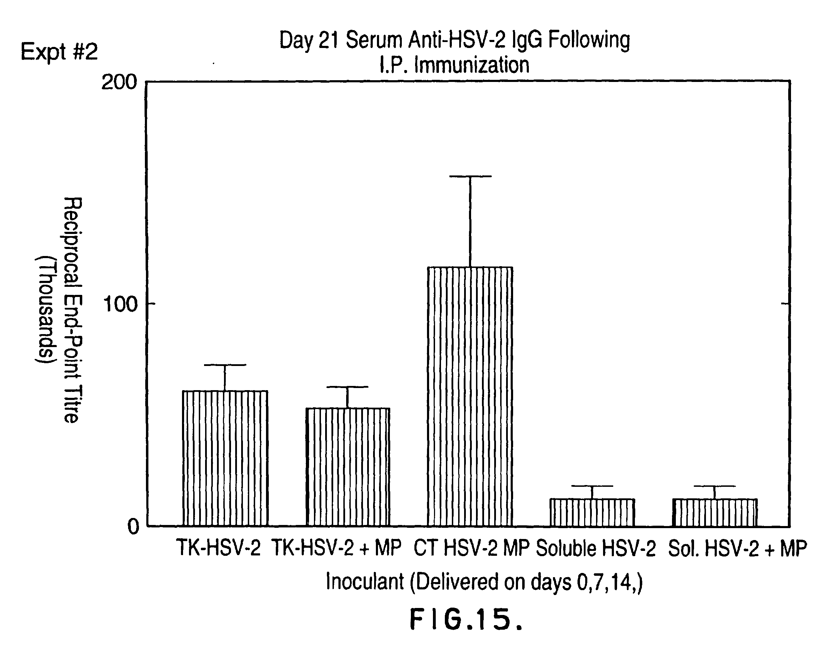

Figure 14 and 15 shows, for two different experiments (Expt #1 and Expt #2 respectively), the anti-HSV-2 antibody titres in the sera of mice immunized by the intraperitoneal route with herpes simplex virus type 2 (HSV-2) infected cell lysate administered in a variety of forms. CT = cholera toxin, UN = uncoated, TK = thymidine kinase.

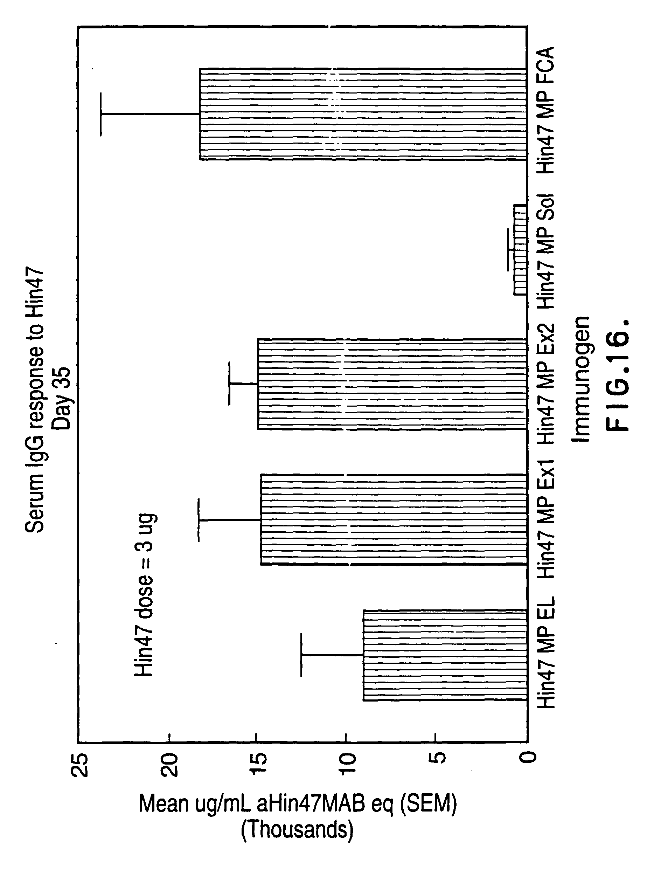

Figure 16 shows the serum IgG responses in mice immunized intraperitoneally by the 47kDa membrane protein from Haemophilus influenzae (Hin47 MP) in a variety of forms. EL = eluate, SOL = soluble, FCA = Freund's Complete Adjuvant, Ex 1 = experiment 1, Ex 2 = experiment 2.

### GENERAL DESCRIPTION OF THE INVENTION

As noted above, the present invention relates to a particulate carrier or microparticle, which is useful for the delivery of biologically-active materials to a vertebrate, generally an animal including humans, including the delivery of antigens to the immune system, by mucosal or parenteral administration.

The particulate carrier comprises two components, namely a solid core and an organometallic polymer bonded to the core.

The solid core comprises at least two components, namely a polysaccharide and a proteinaceous material. The polysaccharide may be one of a wide range of such materials, preferably starch, particularly starch which has been treated as to be "soluble" starch (i.e. a starch which has been treated to provide a starch which is soluble in water). However, other polysaccharide materials may be used, including dextran and cellulose, as well as derivatives and mixtures of two or more polysaccharides.

The particulate carrier may have a particle size which generally ranges from about 10 nm to about 50 µm and preferably about 1 to about 10 µm for mucosal administration of antigens.

The proteinaceous material may be any desired proteinaceous material and may itself have biological activity. Examples of proteinaceous materials which may be used are proteins derived from a variety of viruses and bacteria including tetanus toxoid, diphtheria toxoid, cholera toxoid and subunits thereof, pertussis toxoid, viral subunits, such as rubella virus proteins E1, E2 and C, bacterial subunits, such as the P41, OspA and OspB proteins of B. burgdorferi, protein-polysaccharide conjugates, protozoan subunits, such as T. gondi P30, anticoagulants, venoms, such as snake venom, cytokines, such as interleukins 4, 5, 6 and 12, interferons, tumour necrosis factor, and albumins, such as human serum albumin, bovine serum albumin and ovalbumin, as well as recombinant proteins, peptides and lipopeptides and analogs thereto, including muramyl dipeptide, lipopolysaccharide and lipid A or analogues of such proteins or of immunologic regions of such proteins.

Where the proteinaceous material has biological activity, an additional biologically-active material may or may not be included in the core. Where the proteinaceous material lacks biological activity, a material having biological activity may be incorporated into the core, so that the proteinaceous material acts as a carrier for the biologically-active material.

Both the polysaccharide and proteinaceous material are required to be present for microparticle formation and organometallic polymer coating. In the absence of one of the components, it has not been possible to obtain the particulate carrier of the invention. The proportion of the core comprising proteinaceous material may vary up to about 33 wt% of the core, generally from about 0.5 wt% to about 10 wt%.

Where a biologically-active material is present in the core other than in the form of the proteinaceous material, such material may comprise from about 0.5 to about 30 wt% of the core, preferably from about 0.5 to about 5.0 wt%. Such biologically-active material may be any member of the various classes of known biologically-active materials, including proteins, peptides, antigens, antibodies, immunotargeting molecules, bacteria, bacterial lysates, viruses, virus-infected cell lysates, antibodies, carbohydrates, nucleic acids, lipids, glycolipids, haptens, pharmacologically-active materials, as well as combinations, derivatives and mixtures thereof. Specific examples of such materials include influenza viruses, parainfluenza viruses, respiratory viruses, measles viruses, mumps viruses, human immunodeficiency viruses, polio viruses, rubella viruses, herpex simplex viruses type 1 and 2, hepatitis viruses types A, B and C, yellow fever viruses, smallpox viruses, rabies viruses, vaccinia viruses, reo viruses, rhinoviruses, Coxsackie viruses, Echoviruses, rotaviruses, papilloma viruses, paravoviruses and adenoviruses; E. coli, V. cholera, BCG, C. diphtheria, Y. pestis, S. typhi, B. pertussis, S. aureus, S. pneumoniae, S. pyogenes, S. mutans, Myocoplasmas, Yeasts, C. tetani, meningococci (N. meningitis), Shigella spp, Campylobacter spp, Proteus spp, Neisseria gonorrhoea, and Haemophilus influenzae; as well as proteins obtained from such viruses and bacteria.

The solid core has an organometallic polymer bonded to thereto. Such organometallic compounds may include linear, branched or cross-linked silicones which are bonded at the ends of polymer chains to the core, although the polymer may be bonded to the core at locations along the length of the chain. Such polysiloxanes may vary in molecular weight from about 400 up to about 1,000,000 Daltons and preferably from about 700 to about 60,000 Daltons.

A variety of polysiloxanes may be employed. For the purpose of bonding the polysiloxane to the solid core, the polysiloxanes preferably are derived from functionalized materials which have functional groups at the ends of the polymer chain which facilitate bonding the ends of the polysiloxane chain to the solid core. Preferably, however, where such functional groups are present, they are joined to the polysiloxane chain through end-blocking groups.

Suitable functionalized silicones useful for forming the products of the invention include (trialkoxysilyl) alkyl-terminated polydialkylsiloxanes and trialkoxysilylterminated polydialkylsiloxanes. One useful member of this group of compounds is 3-(triethoxysilyl) propyl-terminated polydimethylsiloxane (herein abbreviated as TS-PDMS).

The organometallic polymer is present in the particulate carrier in relatively minor amounts, generally from about 0.5 to about 5 wt% of the solid core. The presence of the organometallic polymer, particularly a silicone, bonded to the solid core enables biologically-active materials to be administered to a host, particularly by mucosal administration, to achieve an enhanced biological response to such material, for example, an enhanced immune response to an antigen, in comparison to delivery of the material by the same particulate material without the organometallic polymer bonded thereto, as seen from the data presented herein.

The particulate carrier provided herein may be formed in any convenient manner permitting coated particle formation. One preferred procedure is described below with reference to Figure 1.

### DESCRIPTION OF THE PREFERRED EMBODIMENT

Referring to Figure 1, there is shown a method for preparing starch microparticles according to one embodiment of the present invention. Antigen-containing starch microparticles are manufactured by mixing starch and the antigen in solvents, forming an emulsion in oil, and then dispersing the emulsion into an acetone solution with vigorous stirring and collecting the particles formed. The starch or other polysaccharide first is dissolved in a solvent suitable for the polysaccharide. For starch, dimethylsulfoxide is a preferred solvent, in which starch, for example, "soluble" starch, is dissolved at a elevated temperature, for example, a temperature of about 50° to about 100°C, preferably about 75° to about 90°C and then cooled to a lower temperature, particularly to a temperature below about 35°C, without precipitating therefrom. Alternative polar solvents which may be used as solvents for the starch, including dimethylformamide as well as various alcohols.

The starch solution is mixed with an aqueous solution and/or dispersion of a proteinaceous material, in the illustrated embodiment, human serum albumin (HSA), which may be used alone as an antigen or combined with other antigenic material, for example, a herpes simplex virus type 2 (HSV-2) infected cell lysate or a whole influenza strain X31 (Flu X31), in which event the HSA acts also as a carrier protein.

Mixing of the starch solution and antigen composition generally produces by stirring, a highly viscous mixture, which then is added dropwise into vegetable oil, or other water-immiscible fluid which is capable of forming a water-in-oil emulsion, including silicone oils or derivatives thereof or mixtures thereof, with vigorous stirring to promote the formation of a water-in-oil emulsion, in which droplets of the starch-proteinaceous material composition are dispersed in the vegetable oil. This step of the process, therefore, involves forming an emulsion in which the aqueous composition is the dispersed phase.

The particle size of the liquid droplets, which determines the size of the ultimate carrier microparticles, is determined by the volumetric ratio of aqueous phase to oil phase, by the degree of stirring of the water-in-oil emulsion and may further be controlled by sonication. Additional control of particle size may be achieved by employing a surfactant in the oil, such as non-ionic surfactants of the TWEEN or SPAN type.

The water-in-oil emulsion then may be added dropwise to a solvent for the oil and aqueous medium containing the starch, proteinaceous material and antigen, to result in microparticle formation. In the procedure of the present invention, the solvent also contains a silicone polymer material which can bond to the solid core produced by the solvent. Alternatively, some or all the silicone oil can be included in the vegetable oil or silicone oil can replace all or part of the vegetable oil. (Figure 1 also illustrates an alternative procedure, employed in the Examples below to produce particulate carrier lacking the silicone polymer, for comparative experimentation.)

The solvent which may be employed for such desiccation and oil dissolution may be any organic solvent miscible with the oil and water phases of the emulsion and in which the starch and proteinaceous material are substantially insoluble. Such solvents include but are not limited to ketones, such as acetone and methyl ethyl ketone.

The silicone polymer dissolved in the solvent may be a functionalized polysiloxane, particularly end-functionalized, to permit bonding of the polysiloxane to the solid core of the particulate material. Such functionalized polysiloxane may include 3-(trialkoxysilyl) alkyl-terminated polydialkylsiloxanes, particularly 3-triethoxysilyl) propyl-terminated polydimethylpolysiloxane (TS-PDMS).

The resulting particulate material may be harvested from the residual medium by any convenient means, including centrifugation, separated and dried. The particulate material resulting from this procedure then is in a suitable form for formulation for administration of the biologically-active material.

It is clearly apparent to one skilled in the art, that the various embodiments of the present invention have many applications in the fields of medicine and in particular vaccination, diagnosis and treatment of infections with pathogens including bacteria and viruses. A further non-limiting discussion of such uses is further presented below.

### Vaccine Preparation and Use

In an embodiment, immunogenic compositions, suitable to be used as, for example, vaccines, may be prepared from microparticles as disclosed herein. The immunogenic composition elicits an immune response by the host to which it is administered including the production of antibodies by the host.

The immunogenic composition may be prepared as injectables, as liquid solutions or emulsions. The microparticles may be mixed with physiologically acceptable carriers which are compatible with the microparticles. These may include, water, saline, dextrose, glycerol, ethanol and combinations thereof. The vaccine may further contain auxiliary substances such as vetting or emulsifying agents, pH buffering agents, or adjuvants to further enhance the effectiveness of the vaccines. Vaccines may be administered by injection subcutaneously or intramuscularly.

Alternatively, and in a preferred embodiment, the immunogenic compositions comprising microparticles formed according to the present invention, may be delivered in a manner to evoke an immune response at mucosal surfaces. Thus, the immunogenic composition may be administered to mucosal surfaces by, for example, the nasal or oral (intragastric) routes. Alternatively, other modes of administration including suppositories may be desirable. For suppositories, binders and carriers may include, for example, polyalkylene glycols and triglycerides. Oral formulations may include normally employed incipients, such as pharmaceutical grades of saccharin, cellulose and magnesium carbonate.

These compositions may take the form of solutions, suspensions, tablets, pills, capsules, sustained release formulations or powders and contain 1 to 95% of the microparticles of the present invention. In order to protect the microparticles and the material having biological activity contained within the core of the microparticle, from gastric acidity when administered by the oral route, an acidic neutralizing preparation (such as a sodium bicarbonate preparation) is advantageously administered before, concomitant with or directly after administration.

The vaccines are administered in a manner compatible with the dosage formulation, and in such amount as to be therapeutically effective, protective and immunogenic. the quantity to be administered depends on the subject to be treated, including, for example, the capacity of the subject's immune system to synthesize antibodies, and if needed, to produce a cell-mediated immune response. Precise amounts of microparticle and material having biological activity required to be administered depend on the judgement of the practitioner. However, suitable dosage ranges are readily determinable by one skilled in the art and may be of the order of micrograms to milligrams. Suitable regimes for initial administration and booster doses are also variable, but may include an initial administration followed by subsequent administrations. The dosage of the vaccine may also depend on the route of administration and will vary according to the size of the host.

### EXAMPLES

The above disclosure generally describes the present invention. A more complete understanding can be obtained by reference to the following specific Examples. These Examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitations.

### Example 1

This Example describes the production of antigen-containing starch microparticles.

A flow diagram summarising the process of starch microparticle production effected herein is shown in Figure 1. Antigen-containing starch microparticles were manufactured by mixing starch and the antigen in solvents, forming an emulsion in oil, and then dispersing the emulsion into an acetone solution with vigorous stirring and collecting the particles formed. Starch microparticles were separately manufactured containing the antigens, human serum albumin (HSA), tetanus toxoid (TT), ovalbumin (OVA), Hin47, herpes simplex virus type 2 (HSV-2) - infected cell lysate and whole influenza virus. To form the tetanus toxoid, Hin47, HSV-2 and influenza virus-containing starch microparticles, HSA was included as a "filler" protein.

Specifically, 1 g of soluble potato starch was added to 2 mL of dimethylsulfoxide (DMSO) while stirring the mixture. The starch was dissolved by heating the mixture to 85°C for 5 minutes. The following amounts (Table 1) of antigen were prepared to form the antigen-containing microparticles indicated:

**TABLE 1**

| **Antigen entrapped in starch microparticles** | **Antigen Preparation** |
|---|---|
| HSA | 0.1 g of HSA dissolved in 1.0 mL water at room temperature. |
| TT | 70 mg TT and 30 mg HSA dissolved in 1.0 mL H₂O at room temperature. |
| OVA | 100 mg of OVA dissolved in 1.0 mL H₂O at room temperature. |
| Hin47 | 10 mg Hin47 and 90 mg HSA dissolved in 1.0 mL H₂O at room temperature. |
| HSV-2-infected cell-lysate/HSA | 25 mg of HSV-2 in 0.5 mL of buffer and 75 mg HSA in 0.5 mL H₂O. |
| Influenza/HSA | 25 mg Flu X31 in 1 mL 0.1M Tris, 5 mM EDTA pH 7.5, 75 mg HSA in 0.375 mL H₂O. |

When the starch solution had cooled to a temperature of less than 37°C, the antigen preparation indicated above was added to the cooled solution and the mixture stirred (500 rpm) at room temperature for 20 minutes to form a highly viscous mixture. This viscous mixture was added dropwise to 30.0 mL of vegetable oil and stirred vigorously (1500 rpm) for 15 minutes at room temperature to produce a water-in-oil emulsion. This water-in-oil emulsion was sonicated on ice for 60 seconds with stirring. The emulsion was then added dropwise with stirring (1000 rpm) to 400 mL of acetone containing 0.125% v/v Tween 80. The resultant particles, of approximately 4.18 ±3µ were collected by centrifugation, (200 *xg*, 5 minutes), washed twice with acetone and dried by exposure to air at room temperature for 48 hours.

### Example 2

This Example describes the coating of antigen-containing starch microparticles.

The microparticles formed in Example 1 may be coated with a variety of silicones via bonded interactions at the surface including polydimethylsiloxanes (PDMS) with different molecular weights and varied end blocks. A convenient end-functionalized silicone was 3-(triethoxysilyl)propyl-terminated polydimethylsiloxane (abbreviated to TS-PDMS).

The TS-PDMS was synthesised by the hydrosilylative addition of hydrogen-terminated PDMS to allyltriethoxysilane under the catalysis of H₂PtCl₆ as follows. To a mixture of 17.0 mL hydrogen-terminated PDMS (Hüls, PDMS^{H}, viscosity 1,000 CS) and 0.8 mL allyltriethoxysilane (Aldrich) (molar ratio of the functional groups PDMS^{H}: H₂C=CH 1:3) was added 0.05 mL of a 0.1 M hydrogen hexachloroplatinate(IV) hydrate solution (H₂PtCl₆) in *i*-propanol (Caledon) with stirring under the protection of nitrogen at 0°C. The solution was allowed to return to room temperature overnight. The *i*-propanol and unreacted allyltriethoxysilane were evaporated under reduced pressure and elevated temperature up to 140°C for 6 hours until gas ceased to bubble from the viscous fluid. The residue was subjected to further washing with distilled water four times to remove any impurities. The product was characterized by ¹H NMR, ²⁹Si NMR, GPC and IR. The reaction involved is illustrated by the following equation: 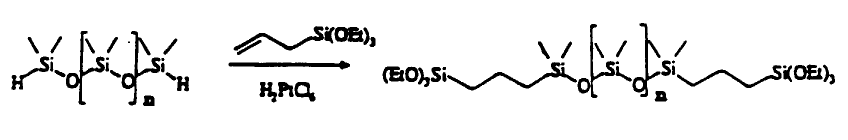 where n is the number of siloxane groups. The use of an end-functionalized silicone resulted in the formation of chemical bonds to the starch surface.

To produce particles coated with TS-PDMS and having antigens entrapped within them, the sonicated water-in-oil emulsion produced by the procedure described above in Example 1 was added dropwise with stirring (1000 rpm) to 400 mL of acetone containing 0.125% v/v TS-PDMS (1,000 c.s.) in place of the Tween 80. The resulting coated particles were harvested and dried as described in Example 1.

### Example 3

This Example describes an analysis of the antigen-containing starch microparticles.

Size distributions of the antigen-containing starch microparticles prepared as described in Examples 1 and 2 were obtained by scanning electron microscopy and flow cytometry using polystyrene microparticle standards. Figure 2 shows a scanning electron microscope analysis of HSA-containing microparticles that were either coated with TS-PDMS or were uncoated. The microparticles ranged in size from 1 to 100 µm and had a mean diameter of 4 to 5 µm as determined by flow cytometry (Figure 3). The efficiency of antigen incorporation into starch microparticles was between 70 and 90%.

The antigen content of HSA-loaded microparticles (termed herein "core loading") was determined by incorporating an ¹²⁵I-HSA tracer of known specific activity in the antigen preparation prior to microparticle formation. Protein core loading of HSA in starch microparticles was found to be about 5 to 6% by weight. The core loading of TT in the microparticle was eliminated by ELISA to be 0.34% w/w with a total protein core loading of 14.1% w/w. The core loading of OVA in the microparticles was estimated to be 7.75% w/w using a spectrophotometer at an O.D.₂₈₀. The core loading of Hin47 in the microparticle was estimated by ELISA to be 0.03% w/w with a total protein core loading of 14% w/w. "Core-loading" of microparticles containing whole influenza virus was thus estimated by the release of virus by degradation of the microparticles by acid hydrolysis with HCl or enzymatic hydrolysis with human saliva.

Enzymatic hydrolysis of microparticles with human saliva was originally the preferred method as it was not anticipated to appreciably alter the antigenic integrity of the viral proteins. Microparticles were digested with 250 µL of centrifugally clarified saliva overnight at 37°C. Suspensions were centrifuged at 5000 *xg* for 10 minutes and the supernatants diluted 1:10 with Tris Base buffered saline (TBS, pH 7.2) containing 0.1% NaN₃ and stored at 4°C until analyzed by SDS-PAGE.

"Core-loading" was determined by acid-hydrolysis of the microparticles. Thus, microparticles were incubated in 0.1 M HCl for 24 hours at 37°C. Supernatants were clarified by centrifugation at 3000 rpm and filtered through a 0.45 µ filter. The solution was neutralized with 1 M NaOH. Protein released from acid hydrolysed microparticles were detected using an ELISA.

The Flu X31/HSA microparticles were estimated to contain about 0.3 to 0.5% of Flu X31 and about 5 to 6% of HSA (w/w). Although HSA may be incorporated into the microparticles preferentially to Flu X31, attempts to fabricate coated microparticles without protein were unsuccessful.

### Example 4

This Example describes the effects upon antigens of their entrapment in starch microparticles.

The time course samples from the antigen release studies described for HSA containing microparticles described in Example 3 were also analyzed by Western (immunoblot) analysis using an HSA-specific polyclonal antiserum. For immunodetection analysis of released HSA, the gel was equilibrated in transfer buffer (0.2 M glycine, 15% methanol, 0.025 M This Base, pH 8.3) for 15 minutes along with nitrocellulose (NC) membranes and filter paper, both of which were cut to the same size as the gel. The immunoblot apparatus was then placed in the transblot device and electrophoretic transfer was performed overnight at 30 volts. After transfer, the NC membrane was incubated with agitation in 100 mL of blocking buffer (5% w/v skim milk powder in PBS) for 2 hours. The NC membrane was then incubated with 100 ml, of a 1:500 dilution of alkaline phosphatase-conjugated goat anti-HSA in blocking buffer for 2 hours at room temperature, on a tilting platform. The NC membrane was washed 3 times (10 minutes each) with PBS, and proteins were visualized by incubating the membrane with 30 mL of developing buffer (100 mM Tris Base, 100 mM NaCl, 5 mM MgCl₂, pH 9.5) containing 200 µL of 50 mg/mL nitroblue tetrazolium and 100 µL of 50 mg/mL 5-bromo-4-chloro-3-indolylphosphate for 60 minutes. The membrane was rinsed 3 times with H₂O and air dried. The results of the immunoblot analysis are shown in Figure 4. This analysis showed that HSA released into the supernatants by HCl treatment or incubation of the microparticles in PBS was detectable by an HSA-specific polyclonal antiserum. The released HSA from uncoated and TS-PDMS coated microparticles, was not fragmented by the fabrication process and was not altered in such a way as to preclude its detection by HSA-specific antibodies.

### Example 5

This Example describes the immunogenicity of HSA entrapped in microparticles in mice immunised intraperitoneally.

To examine the immunogenicity of HSA entrapped in starch microparticles formed in accordance with the present invention, groups of six, 6 to 8 week old female BALB/c mice (Charles River Breeding Laboratories, Wilmington, MA) were immunized intraperitoneally (IP) with the following amounts of antigen in 250 µL of PBS (pH 7.4) on days 0, 7 and 14: 2 mg of TS-PDMS coated microparticles prepared as described in Examples 1 and 2 containing 100 µg of HSA; and 2 mg of uncoated microparticles containing 100 µg of HSA.

The mice showed no gross pathologies or behaviourial changes after receiving either uncoated or TS-PDMS coated microparticles. Sara were obtained on days +21, +35, +49, +63 and +84 and were evaluated for the presence of anti-HSA IgG antibodies by antigen specific ELISA. All samples were analyzed in duplicate. Microtiter plate wells were incubated overnight at 4°C with 100 µL of 10 µg/mL HSA in TBS. The plates were washed with Tris-T buffer (0.05% Tween 20 in 0.02 M Tris Base, pH 7.4, containing 0.15 M NaCl and 0.005 M KCl). Wells were incubated with 200 µL of 0.1% gelatin in 0.02 M Tris-buffered saline (TBS), pH 7.4 (operationally defined as blocking buffer). After washing with Tris-T, the plates were incubated for 2 h at 37°C with 100 µL of sample serially diluted in blocking buffer. Wells were washed with Tris-T and 100 µL of alkaline phosphatase-conjugated goat anti-mouse IgG in blocking buffer, were added to each well. After 2 hours incubation at 37°C, the wells were washed with Tris-T and 100 µL of 1.0 M diethanolamine buffer, pH 9.8, containing 0.05 M MgCl₂ and 1.0 mg/mL of p-nitrophenylphosphate were added to each well. After 30 minutes incubation at room temperature, the optical density of the fluid in each well was determined at 405 nm using a microplate reader. A normal mouse sera pool was used to establish baseline optical density values in the assay. Hyperimmune mouse HSA antiserum was used as a positive control.

The serum antibody titres following immunization are shown in Figure 5. The results of immunizations with a convenient test antigen (HSA) indicate that antigen presented to the immune system entrapped in TS-PDMS starch microparticles is substantially more immunogenic than soluble antigen or antigen entrapped in uncoated starch microparticles.

### Example 6

This Example describes the immunogenicity of HSA entrapped in starch microparticles in mice immunized by the intragastric route.

To examine the immunogenicity of HSA entrapped in starch microparticles formed in accordance with the present invention, groups of six, 6 to 8 week old female BALB/c mice, were immunized by the intragastric route (IG) with HSA-containing microparticles, prepared as described in Examples 1 (uncoated) and 2 (coated) above, (Table II) on days 0 +7 and +14:

**TABLE II**

| Group: | Microparticle ¹ Type: | mg particle: | µg HSA: |
|---|---|---|---|
| A | TS-PDMS coated | 15 | 750 |
| B | TS-PDMS coated | 10 | 500 |
| C | TS-PDMS coated | 3 | 150 |
| D | TS-PDMS coated | 1.5 | 75 |
| E | TS-PDMS coated | 1 | 50 |
| F | uncoated | 15 | 750 |
| G | uncoated | 10 | 500 |
| H | uncoated | 3 | 150 |
| I | uncoated | 1.5 | 75 |
| J | uncoated | 1 | 50 |
| K | none | 0 | 0 |
| N | none | - | - |
| O | none | - | 750 |
| P | none | - | 500 |
| Q | none | - | 150 |
| R | none | - | 75 |
| S | none | - | 50 |
| 1 mg of TS-PDMS coated microparticle contains 50 µg of HSA. | | | |

Sera were examined for the presence of HSA-specific antibodies on days +21, +35 and +49.

Sera and intestinal washes were examined for the presence of HSA-specific antibodies. To detect and quantify anti-HSA sIgA in the intestinal lumen, mice were sacrificed by cervical dislocation, their small intestines removed and examined for the presence of antigen-specific antibodies. Individual small intestines were detached from the pyloric sphincter to the caecum and everted over capillary tubes. The everted intestines were incubated in 5 mL of ice cold enzyme inhibitor solution (0.15 M NaCl, 0.01 M Na₂HPO₄, 0.005 M EDTA, 0.002 M PMSF, 0.05 U/mL Aprotinin, and 0.02% v/v NaN₃) for 4 hours. Intestines were removed and the supernatants clarified by centrifugation (1000 *xg*, 20 minutes) and stored at 0°C until assayed. Anti-HSA sIgA titres in samples were determined by HSA-specific ELISA as described above but a goat anti-mouse IgA antiserum was used in place of the goat anti-mouse IgG antiserum.

The percentage of mice immunologically responding to the intragastric immunization is shown in Figure 6. These results show that a much higher proportion of animals immunologically respond to the test antigen (HSA) when delivered using PDMS-coated microparticles compared to uncoated microparticles at physiologically relevant doses, for example, 75 µg or less.

The serum IgG HSA-specific antibody titres following IG immunization are shown in Figures 7 (50 µg of HSA) and 8 (75 µg of HSA). These results indicate that a test antigen (HSA) incorporated into PDMS-coated microparticles is substantially more immunogenic than antigen incorporated into uncoated particles when delivered by the intragastric route.

### Example 7

The procedure of Example 6 was repeated with groups of 15 mice being immunized intragastrically with 50 µg and 10 µg of aminoencapsulated HSA, prepared as described in Example 1 (uncoated) and Example 2 (coated) above. Animals were immunized on days 0, 7, 14 and 70 with the HSA-containing microcapsules (MP). Sera was examined for the presence of HSA-specific antibodies on days 35, 49, 63 and 77. The results for IgG (Panels A and C) and IgA (Panels B and D) responses are given in Figure 4.

Figure 4 shows that, at various times after tertiary IG immunization, anti-HSA IgG sera titres induced by IG administration of 50 µg (Panels A and B) or 10 µg (Panel C) of HSA-containing TS-PDMS-grafted MP (solid bars) were significantly higher when compared to IgG responses elicited following immunization with ungrafted MP (hatched bars) (p<0.005). Sera IgG induced by IG immunization were almost exclusively IgG, with very little IgG₂ₐ or IgG_{2b} and no IgG₃ antibodies.

Further, IG administration of 50 µg (Panel B) or 10 µg (Panel D) of HSA in TS-PDMS-grafted MP, stimulated stronger anti-HSA sera IgA responses, with sera IgA responses being significantly higher when animals were immunized with 50 µg of HSA-containing ungrafted MP (P<0.001). At all times, animals immunized IG with soluble HSA failed to produce any detectable anti-HSA sera IgG or IgA.

Following an IG boost on day 70, anti-HSA sera IgG titres induced with 50 µg of HSA contained in TS-PDMS-grafted MP were significantly enhanced over pre-boost titres (P<0.001). These results demonstrate the efficacy of IG immunization with TS-PDMS MP in stimulating vigorous circulating antibody response.

In contrast to the failure of soluble HSA to provoke an appreciable IgA response in intestinal secretions when administered IG, the delivery of equal amounts of HSA entrapped in TS-PDMS-grafted or ungrafted MP resulted in HSA-specific IgA responses in gut secretions (P<0.001).

### Example 8

This Example describes the immunogenicity of herpes simplex type 2 virus (HSV-2) antigens entrapped in microparticles in mice immunized by the intraperitoneal and intragastric routes.

To examine the stimulation of virus-specific immune responses by viral antigens entrapped in microparticles, mice were immunized IP and IG with HSV-2 infected cell lysates entrapped within TS-PDMS coated microparticles containing HSA as a carrier protein. Groups of 5, 6-8 week old female BALB/c mice were immunized by the intraperitoneal (IP) and intragastric (IG) routes with the following materials on days 0, +7 and +14:
1. 125 µg of HSV-2 infected cell lysate protein in 250 µL of PBS (IP) or 500 µL of NaHCO₃ (IG).
2. 16 mg of TS-PDMS coated microparticles containing about 125 µg of HSV-2 infected cell lysate.
3. 8 mg of TS-PDMS coated microparticles containing about 63 µg of HSV-2 infected cell lysate protein.

Sera were examined for the presence of HSV-2 specific IgG antibodies and demonstrated that viral proteins may be entrapped within TS-PDMS coated starch microparticles without reduction in immunogenicity.

### Example 9

This Example describes the immunogenicity of whole influenza virus entrapped in microparticles in mice immunized IP.

To examine the immunogenicity of Flu X31/HSA TS-PDMS coated microparticles, prepared as described in Example 2, groups of six Balb/c mice were immunized by intraperitoneal (IP) route with the following materials:
1. 5 µg of Flu X31 and 15 µg of HSA in soluble form.
2. 5 µg of Flu X31 and 15 µg of HSA mixed with TS-PDMS coated microparticles.
3. Flu X31/HSA TS-PDMS coated microparticles containing 5 µg of Flu X31 and 15 µg of HSA.

The mice received a single immunization IP on day 0 and were bled at days +20 and +35. The sera obtained were assayed for anti-Flu X31 and anti-HSA IgG antibodies by antigen-specific ELISA. The anti-Flu X31 ELISA was performed as described above but the plates were coated overnight at 4°C with 100 µL of whole influenza virus at 5 µg per mL in place of the HSA and an anti-Flu antibody was used as a positive control. These antibody titres are shown in Figures 10 and 11 for Flu X31 and HSA immunized mice respectively.

As described in Example 5 above, HSA alone or HSA mixed with TS-PDMS coated microparticles were poorly immunogenic. In contrast, HSA entrapped in TS-PDMS coated microparticles elicited high antibody titres.

Mice immunized IP with all three preparations showed similar serum IgG anti-Flu X31 antibody responses on day +20. At day +35 the IgG anti-Flu X31 antibody titre in the serum of mice immunized IP with Flu X31/HSA incorporated in TS-PDMS coated microparticles was about 10-fold greater than the titres obtained following immunization with soluble Flu X31 or Flu X31 mixed with TS-PDMS coated microparticles.

The studies presented in this Example demonstrate that viral antigens from influenza virus can be made more immunogenic and elicit high levels of serum IgG antibodies, when the antigens are entrapped in microparticles formed in accordance with the present invention.

### Example 10

This Example describes the immunogenicity of whole influenza virus entrapped in microparticles in mice immunized IN.

To examine the immunogenicity of Flu X31/HSA TS-PDMS coated microparticles, prepared as described in Example 2, groups of six Balb/c mice were immunized by the intranasal (IN) route with the following materials:
1. 10 µg of Flu X31 and 30 µg of HSA in soluble form.
2. Flu X31/HSA TS-PDMS coated microparticles containing 10 µg of Flu X31 and 30 µg of HSA.

Mice were immunized IN on days 0 +7 and +14 and bled on days +20 and +35. The sera obtained were assayed for anti-Flu X31 and anti-HSA IgG antibodies by antigen-specific ELISA as described above. These serum antibody titres are shown in Figures 12 and 13 for HSA and Flu X31 respectively.

Mice immunized IN with soluble antigen had undetectable levels of HSA-specific serum IgG antibodies. Mice immunized with Flu X31/HSA TS-PDMS coated microparticles showed a serum anti-HSA antibody response.

The anti-Flu X31 antibody titres in mice immunized IN are shown in Figure 13 and show that the highest titres were obtained following immunization with Flu X31/HSA TS-PDMS coated microparticles.

The results of the IN immunizations described in this Example show that the immunogenicity of an antigen (HSA) and a mixture of influenza virus antigens can be enhanced by entrapment in microparticles formed in accordance with the present invention. In particular, the normally non-immunogenic antigen HSA following incorporation into microparticles was made immunogenic.

### Example 11

This Example describes the immunogenicity of HSV-2 entrapped in microparticles in mice immunized intragastrically (IG).

TS-PDMS-grafted (CT) or ungrafted (UN) microparticles (MP) were fabricated to contain HSV-2-infected VERO cell lysate and HSA, as described in Examples 1 and 2. In a first set of experiments, using the core loading data as determined in Example 2, groups of 10 mice each were immunized intragastrically (IG) on days 0, 7, 14 and 77 with 25 µg of HSV-2 infected cell lysate entrapped in grafted or ungrafted MP (CT HSV-2 MP and UN HSV-2 MP respectively), 25 µg of HSV-2 infected cell lysate suspended in buffer (Soluble HSV-2) or buffer alone (Buffer). Mice were bled on day 77 via the retroorbital plexus and their sera assayed for the presence of HSV-2 specific IgG. The results obtained are expressed as reciprocal end-point titres and illustrated in Figure 14. ("Expt. 1").

As may be seen in this Figure, HSV-2-infected cell lysate entrapped in CT MP was significantly more proficient at stimulating HSV-2-specific IgG antibodies than HSV-2-entrapped UN MP or soluble HSV-2.

In a second set of experiments, groups of 10 mice were immunized intraperitoneally (IP) on days 0, 7 and 14 with one of the following preparations:
- 5 µg of HSV-2-infected cell lysate entrapped in CT MP (CT HSV-2 MP)
- 5 µg of soluble HSV-2 dissolved in buffer (Soluble HSV-2)
- 5 µg soluble HSV-2 dissolved in buffer and mixed with HSA-containing CT MP (Sol. HSV-2 + MP)
- 1 x 10⁵, plaque-forming units (PFU) of ΔTK⁻HSV-2 (an attenuated, non-lethal HSV-2 mutant which is highly immunogenic) (TK⁻HSV-2)
- 1 x 10⁵ PFU ΔTK⁻HSV-2 mixed with HSA-containing CT MP (TK-HSV-2 + MP).
On day 21, the mice were bled and their sera assayed for HSV-2-specific IgG antibodies. The results obtained are expressed as reciprocal end-point titres and illustrated in Figure 15 ("Expt. 2").

As may be seen from these results, CT HSV-2 MP elicited the strongest HSV-2-specific IgG antibody responses, higher than stimulated by the "gold standard", ΔTK⁻HSV-2. Mixing HSA CT MP and either ΔTK⁻HSV-2 or soluble HSV-2 neither enhanced nor diminished the observed antibody response, demonstrating that the immunopotentiating effect of encapsulated HSV-2 required entrapment of antigen inside particles.

### Example 12

This Example describes the immunogenicity of Hin47 entrapped in microparticles in mice immunized intraperitoneally (IP).

Microparticles containing Hin47 antigen were prepared as described in Example 2. Using the Hin47 core loading data as determined in Example 2, groups of 6 to 10 mice were given intraperitoneal injections of 3 µg per mouse on days 0, 7 and 14. Mice were bled via the retrobital plexus on day 5 and their sera was assayed for anti-Hin47 IgG. The results obtained are shown in Figure 13.

As may be seen in Figure 16, soluble (3 µg) Hin47 in buffer (Hin47 sol) elicited IgG responses of approximately 1000 units. Hin47 (3 µg) in conjunction with FCA produced IgG responses of approximately 18,000 units. By comparison, Hin47 in silicone-grafted microparticles (Hin47 MP Ex 1 and Hin47 MP Ex. 2) elicited responses of about 15,000 units, i.e. about 84% of that noted with FCA.

In another experiment, the Hin47 microparticles were extracted with buffer *in vitro* for 18 hours at 37°C, clarified by centrifugation and filtration and 3 µg of Hin47 contained in the extract was administered IP. As seen in Figure 16, this preparation (Hin47 MP EL) elicited IgG responses of approximately 9,000 units.

### SUMMARY OF THE DISCLOSURE

In summary of this disclosure, the present invention provides a particulate carrier for an agent, particularly one having biological activity, comprising a core of polysaccharide and proteinaceous material and an organometallic polymer bonded to the core. The particulate carriers in the form of microparticles are able to efficiently deliver agents to the cells of the immune system of a subject following mucosal or parenteral administration to produce an immune response.

## Claims

1. A particulate carrier for delivering materials having biological activity to a host,characterized by:
a solid core comprising a polysaccharide and a proteinaceous material, and
an organometallic polymer bonded to the core.

2. The particulate carrier claimed in claim 1, wherein the polysaccharide is selected from the group consisting of dextran, starch, cellulose, derivatives and mixtures thereof.

3. The particulate carrier claimed in claim 1, wherein the polysaccharide is a soluble starch.

4. The particulate carrier claimed in any one of claims 1 to 3, wherein the proteinaceous material is a material having biological activity.

5. The particulate carrier claimed in any one of claims 1 to 4, wherein said core contains a material having biological activity.

6. The particulate carrier claimed in claim 5, wherein the material having biological activity is selected from the group consisting of proteins, peptides, antigens, bacteria, bacterial lysates, viruses, virus-infected cell lysates, antibodies, carbohydrates, nucleic acids, lipids, glycolipids haptens, pharmacologically-active materials, and combinations, derivatives and mixtures thereof.

7. The particulate carrier claimed in any one of claims 4 to 6, wherein the material having biological activity is immunogenic.

8. The particulate carrier claimed in any one of claims 4 to 7, wherein the material having biological activity comprises human serum albumin, herpes simplex virus type 2 - infected cell lysate, an influenza virus, or an influenza viral protein.

9. The particulate carrier claimed in any one of claims 5 to 8, wherein said biologically-active material is present in an amount of from 0.5 to 30 wt% of the core.

10. The particulate carrier claimed in claim 9, wherein said biologically-active material comprises about 0.5 to about 5.0 wt% of the core.

11. The particulate carrier claimed in any one of claims 1 to 10 wherein the organometallic polymer is derived from a functionalized silicone.

12. The particulate carrier claimed in claim 11, wherein the functionalized silicone comprises an end-substituted silicone.

13. The particulate carrier claimed in claim 12, wherein the, end-substituted silicone is (trialkoxysilyl)alkyl-terminated polydialkylsiloxane.

14. The particulate carrier claimed in claim 13, wherein the end-substituted silicone is 3-(triethoxysilyl)propyl-terminated polydimethylsiloxane.

15. The particulate carrier claimed in any one of claims 11 to 14, wherein said silicone has a molecular weight of from 400 to 1,000,000 Daltons.

16. The particulate carrier of claim 15, wherein said silicone is a polysiloxane having a molecular weight of from 700 to 60,000 Daltons.

17. The particulate carrier claimed in any one of claims 1 to 15, wherein said solid core has a particle size of 10 nm to 50 µm comprising a polysaccharide and up to 33 wt% of a proteinaceous material, and said organometallic polymer is in an amount of 0.5 to 5 wt% of said core bonded to the core.

18. The particulate carrier claimed in claim 17, wherein said proteinaceous material comprises from 0.5 to 10 wt% of said core.

19. The particulate carrier claimed in any one of claims 1 to 18 which has a particle size of 1 to 10 µm.

20. A method for producing a particulate carrier for delivering materials having biological activity to a host, characterized by:
(a) forming an aqueous composition comprising a dissolved polysaccharide and a dispersed or dissolved proteinaceous material;
(b) forming an emulsion in which the aqueous composition is the dispersed phase;
(c) forming from said emulsion a particulate carrier comprising a core of said polysaccharide and proteinaceous material having bonded thereto an organometallic polymer; and
(d) collecting the particulate carrier so formed.

21. The method claimed in claim 21, wherein said aqueous composition is formed by dissolving said polysaccharide in a polar solvent therefor to form a solution thereof, dissolving or dispersing said proteinaceous material in an aqueous solvent therefor to form a solution or dispersion thereof, and mixing the resulting media.

22. The method claimed in claim 21, wherein said polysaccharide is starch and said solvent for said starch is dimethylsulfoxide.

23. The method claimed in any one of claims 20 to 22, wherein said emulsion is formed by dispersing said aqueous composition in a water-immiscible fluid capable of forming a water-in-oil emulsion.

24. The method claimed in claim 23, wherein said water-immiscible fluid comprises a vegetable oil.

25. The method claimed in claim 23 or 24, wherein said water-in-oil emulsion also contains a surfactant.

26. The method claimed in any one of claims 23 to 25, wherein said particulate carrier is formed by adding said water-in-oil emulsion dropwise to a solvent for water and said water-immiscible fluid containing a functionalized organometallic polymer.

27. The method claimed in claim 26, wherein said functionalized organometallic polymer comprises an end-substituted silicone.

28. The method claimed in claim 26 or 27, wherein said solvent comprises a ketone.

29. The method claimed in claim 28 wherein said ketone is acetone.

30. The method claimed in any one of claims 20 to 29 which is carried out under conditions which are not conducive to denaturation of said proteinaceous material.

31. An immunogenic composition formulated for mucosal or parenteral administration, comprising the particulate carrier claimed in claim 7 or 8 and a physiological acceptable carrier therefor.

32. A particulate carrier comprising a solid core comprising a polysaccharide and a proteinaceous material, and an organometallic polymer bonded to the core for use in the delivery of materials having biological activity in a host.

33. The use of a particulate carrier comprising a solid core comprising polysaccharide, a proteinaceous material and a material having biological activity, and an organometallic polymer bonded to the core in the manufacture of an immunogenic composition for delivery of the material, having biological activity to a host.

## Patentansprüche

1. Teilchenförmiger Träger zum Abgeben von Materialien mit biologischer Aktivität an einen Wirt, gekennzeichnet durch:
einen festen Kern, der ein Polysaccharid und ein proteinhaltiges Material umfaßt, und
ein organometallisches Polymer, das an den Kern gebunden ist.

2. Teilchenförmiger Träger nach Anspruch 1, wobei das Polysaccharid aus der Gruppe, bestehend aus Dextran, Stärke, Cellulose, Derivaten und Mischungen davon, ausgewählt ist.

3. Teilchenförmiger Träger nach Anspruch 1, wobei das Polysaccharid lösliche Stärke ist.

4. Teilchenförmiger Träger nach einem der Ansprüche 1 bis 3, wobei das proteinhaltige Material ein Material mit biologischer Aktivität ist.

5. Teilchenförmiger Träger nach einem der Ansprüche 1 bis 4, wobei der Kern ein Material mit biologischer Aktivität enthält.

6. Teilchenförmiger Träger nach Anspruch 5, wobei das Material mit biologischer Aktivität aus der Gruppe, bestehend aus Proteinen, Peptiden, Antigenen, Bakterien, Bakterienlysaten, Viren, Lysaten virusinfizierter Zellen, Antikörpern, Kohlenhydraten, Nukleinsäuren, Lipiden, Glykolipiden, Haptenen, pharmakologisch wirksamen Materialien und Kombinationen, Derivaten und Mischungen davon, ausgewählt ist.

7. Teilchenförmiger Träger nach einem der Ansprüche 4 bis 6, wobei das Material mit biologischer Aktivität immunogen ist.

8. Teilchenförmiger Träger nach einem der Ansprüche 4 bis 7, wobei das Material mit biologischer Aktivität menschliches Serumalbumin, Lysate von durch Herpes simplex-Virus Typ 2 infizierten Zellen, ein Influenza-Virus oder ein Influenza-Virus-Protein umfaßt.

9. Teilchenförmiger Träger nach einem der Ansprüche 5 bis 8, wobei das biologisch aktive Material in einer Menge von 0,5 bis 30 Gew.-% des Kerns vorliegt.

10. Teilchenförmiger Träger nach Anspruch 9, wobei das biologisch aktive Material ungefähr 0,5 bis ungefähr 5,0 Gew.-% des Kerns umfaßt.

11. Teilchenförmiger Träger nach einem der Ansprüche 1 bis 10, wobei das organometallische Polymer von einem funktionalisierten Silicon abgeleitet ist.

12. Teilchenförmiger Träger nach Anspruch 11, wobei das funktionalisierte Silicon ein endsubstituiertes Silicon umfaßt.

13. Teilchenförmiger Träger nach Anspruch 12, wobei das endsubstituierte Silicon (Trialkoxysilyl)alkyl-terminiertes Polydialkylsiloxan ist.

14. Teilchenförmiger Träger nach Anspruch 13, wobei das endsubstituierte Silicon 3-(Triethoxysilyl)propyl-terminiertes Polydimethylsiloxan ist.

15. Teilchenförmiger Träger nach einem der Ansprüche 11 bis 14, wobei das Silicon eine relative Molekülmasse von 400 bis 1000000 Dalton hat.

16. Teilchenförmiger Träger nach Anspruch 15, wobei das Polysiloxan eine relative Molekülmasse von 700 bis 60000 Dalton hat.

17. Teilchenförmiger Träger nach einem der Ansprüche 1 bis 15, wobei der feste Kern eine Teilchengröße von 10 nm bis 50 µm aufweist, wobei er ein Polysaccharid und bis zu 33 Gew.-% eines proteinhaltigen Materials umfaßt, und das organometallische Polymer in einer Menge von 0,5 bis 5 Gew.-% des Kerns an den Kern gebunden vorliegt.

18. Teilchenförmiger Träger nach Anspruch 17, wobei das proteinhaltige Material 0,5 bis 10 Gew.-% des Kerns umfaßt.

19. Teilchenförmiger Träger nach einem der Ansprüche 1 bis 18, der eine Teilchengröße von 1 bis 10 µm hat.

20. Verfahren zum Herstellen eines teilchenförmigen Trägers zum Abgeben von Materialien mit biologischer Aktivität an einen Wirt, gekennzeichnet durch:
(a) Bilden einer wäßrigen Zusammensetzung, die ein gelöstes Polysaccharid und ein dispergiertes oder gelöstes proteinhaltiges Material umfaßt;
(b) Bilden einer Emulsion, in der die wäßrige Zusammensetzung die dispergierte Phase darstellt;
(c) Bilden eines teilchenförmigen Trägers, der einen Kern aus dem Polysaccharid und dem proteinhaltigen Material, an den ein organometallisches Polymer gebunden ist, umfaßt, aus der Emulsion; und
(d) Sammeln des so gebildeten teilchenförmigen Trägers.

21. Verfahren nach Anspruch 21, wobei die wäßrige Zusammensetzung durch Lösen des Polysaccharids in einem polaren Lösemittel dafür zur Bildung einer Lösung desselben, Lösen oder Dispergieren des proteinhaltigen Materials in einem wäßrigen Lösemittel dafür zur Bildung einer Lösung oder Dispersion desselben und Mischen der resultierenden Medien gebildet wird.

22. Verfahren nach Anspruch 21, wobei das Polysaccharid Stärke und das Lösemittel für die Stärke Dimethylsulfoxid ist.

23. Verfahren nach einem der Ansprüche 20 bis 22, wobei die Emulsion durch Dispergieren der wäßrigen Zusammensetzung in einem mit Wasser nicht mischbaren Fluid, das in der Lage ist, eine Wasser-in-Öl-Emulsion zu bilden, gebildet wird.

24. Verfahren nach Anspruch 23, wobei das mit Wasser nicht mischbare Fluid ein pflanzliches Öl umfaßt.

25. Verfahren nach Anspruch 23 oder 24, wobei die Wasser-in-Öl-Emulsion auch ein grenzflächenaktives Mittel enthält.

26. Verfahren nach einem der Ansprüche 23 bis 25, wobei der teilchenförmige Träger durch tropfenweises Zugeben der Wasser-in-Öl-Emulsion zu einem Lösemittel für Wasser und dem mit Wasser nicht mischbaren Fluid, das ein funktionalisiertes organometallisches Polymer enthält, gebildet wird.

27. Verfahren nach Anspruch 26, wobei das funktionalisierte organometallische Polymer ein endsubstituiertes Silicon umfaßt.

28. Verfahren nach Anspruch 26 oder 27, wobei das Lösemittel ein Keton umfaßt.

29. Verfahren nach Anspruch 28, wobei das Keton Aceton ist.

30. Verfahren nach einem der Ansprüche 20 bis 29, das unter Bedingungen, die einer Denaturierung des proteinhaltigen Materials nicht förderlich sind, ausgeführt wird.

31. Immunogene Zusammensetzung, die für eine Verabreichung an Schleimhäute oder eine parenterale Verabreichung formuliert ist und den teilchenförmigen Träger nach Anspruch 7 oder 8 und einen physiologisch annehmbaren Träger dafür umfaßt.

32. Teilchenförmiger Träger, der einen festen Kern, umfassend ein Polysaccharid und ein proteinhaltiges Material, und ein organometallisches Polymer, das an den Kern gebunden ist, umfaßt, für eine Verwendung bei der Abgabe von Materialien mit biologischer Aktivität in einem Wirt.

33. Verwendung eines teilchenförmigen Trägers, der einen festen Kern, umfassend Polysaccharid, ein proteinhaltiges Material und ein Material mit biologischer Aktivität, und ein organometallisches Polymer, das an den Kern gebunden ist, umfaßt, bei der Herstellung einer immunogenen Zusammensetzung für eine Abgabe des Materials mit biologischer Aktivität an einen Wirt.

## Revendications

1. Support particulaire pour fournir des substances ayant une activité biologique à un hôte, caractérisé par :
un noyau solide comprenant un polysaccharide et une substance protéique, et
un polymère organométallique lié au noyau.

2. Support particulaire selon la revendication 1, dans lequel le polysaccharide est choisi dans le groupe consistant en le dextrane, l'amidon, la cellulose, leurs dérivés et leurs mélanges.

3. Support particulaire selon la revendication 1, dans lequel le polysaccharide est un amidon soluble.

4. Support particulaire selon l'une quelconque des revendications 1 à 3, dans lequel la substance protéique est une substance ayant une activité biologique.

5. Support particulaire selon l'une quelconque des revendications 1 à 4, dans lequel ledit noyau contient une substance ayant une activité biologique.

6. Support particulaire selon la revendication 5, dans lequel la substance ayant une activité biologique est choisie dans le groupe consistant en les protéines, les peptides, les antigènes, les bactéries, les lysats bactériens, les virus, les lysais cellulaires infectés par des virus, les anticorps, les glucides, les acides nucléiques, les lipides, les glycolipides, les haptènes, les substances pharmacologiquement actives et leurs combinaisons, leurs dérivés et leurs mélanges.

7. Support particulaire selon l'une quelconque des revendications 4 à 6, dans lequel la substance ayant une activité biologique est immunogène.

8. Support particulaire selon l'une quelconque des revendications 4 à 7, dans lequel la substance ayant une activité biologique comprend de la sérumalbumine humaine, un lysat cellulaire infecté par l'herpèsvirus de type 2, un virus de la grippe ou une protéine virale de la grippe.

9. Support particulaire selon l'une quelconque des revendications 5 à 8, dans lequel ladite substance biologiquement active est présente en une quantité de 0,5 à 30 % en masse du noyau.

10. Support particulaire selon la revendication 9, dans lequel ladite substance biologiquement active constitue environ 0,5 à 5,0 % en masse du noyau.

11. Support particulaire selon l'une quelconque des revendications 1 à 10, dans lequel le polymère organométallique est dérivé d'un silicone fonctionnalisé.

12. Support particulaire selon la revendication 11, dans lequel le silicone fonctionnalisé comprend un silicone substitué aux extrémités.

13. Support particulaire selon la revendication 12, dans lequel le silicone substitué aux extrémités est un polydialkylsiloxane à terminaisons (trialcoxysilyl)alkyle.

14. Support particulaire selon la revendication 13, dans lequel le silicone substitué aux extrémités est un polydiméthylsiloxane à terminaisons 3-(triéthoxysilyl)propyle.

15. Support particulaire selon l'une quelconque des revendications 11 à 14, dans lequel ledit silicone a une masse moléculaire de 400 à 1 000 000 daltons.

16. Support particulaire selon la revendication 15, dans lequel ledit polysiloxane a une masse moléculaire de 700 à 60 000 daltons.

17. Support particulaire selon l'une quelconque des revendications 1 à 15, dans lequel ledit noyau solide a une taille de particule de 10 nm à 50 µm comprenant un polysaccharide et jusqu'à 33 % en masse d'une substance protéique, et ledit polymère organométallique est lié au noyau en une quantité de 0,5 à 5 % en masse dudit noyau.

18. Support particulaire selon la revendication 17, dans lequel ladite substance protéique constitue de 0,5 à 10 % en masse dudit noyau.

19. Support particulaire selon l'une quelconque des revendications 1 à 18, qui a une taille de particule de 1 à 10 µm.

20. Procédé de production d'un support particulaire pour fournir des substances ayant une activité biologique à un hôte, caractérisé par :
(a) la formation d'une composition aqueuse comprenant un polysaccharide dissous et une substance protéique dispersée ou dissoute ;
(b) la formation d'une émulsion dans laquelle la composition aqueuse est la phase dispersée ;
(c) la formation à partir de ladite émulsion d'un support particulaire comprenant un noyau dudit polysaccharide et de ladite substance protéique auquel est lié un polymère organométallique ; et
(d) la récupération du support particulaire ainsi formé.

21. Procédé selon la revendication 21, dans lequel ladite composition aqueuse est formée par dissolution dudit polysaccharide dans un solvant polaire pour celui-ci pour former une solution de celui-ci, la dissolution ou la dispersion de ladite substance protéique dans un solvant aqueux pour celle-ci pour former une solution ou une dispersion de celle-ci, et le mélange des milieux résultants.

22. Procédé selon la revendication 21, dans lequel ledit polysaccharide est l'amidon et ledit solvant pour ledit amidon est le diméthylsulfoxyde.

23. Procédé selon l'une quelconque des revendications 20 à 22, dans lequel ladite émulsion est formée par dispersion de ladite composition aqueuse dans un fluide immiscible à l'eau capable de former une émulsion eau dans l'huile.

24. Procédé selon la revendication 23, dans lequel ledit fluide immiscible à l'eau comprend une huile végétale.

25. Procédé selon la revendication 23 ou 24, dans lequel ladite émulsion eau dans l'huile contient aussi un tensioactif.

26. Procédé selon l'une quelconque des revendications 23 à 25, dans lequel ledit support particulaire est formé par addition goutte à goutte de ladite émulsion eau dans l'huile à un solvant pour l'eau et ledit fluide immiscible à l'eau contenant un polymère organométallique fonctionnalisé.

27. Procédé selon la revendication 26, dans lequel ledit polymère organométallique fonctionnalisé comprend un silicone substitué aux extrémités.

28. Procédé selon la revendication 26 ou 27, dans lequel ledit solvant comprend une cétone.

29. Procédé selon la revendication 28, dans lequel ladite cétone est l'acétone.

30. Procédé selon l'une quelconque des revendications 20 à 29, qui est mis en oeuvre dans des conditions qui ne conduisent pas à une dénaturation de ladite substance protéique.

31. Composition immunogène formulée pour l'administration mucosale ou parentérale, comprenant le support particulaire selon la revendication 7 ou 8 et un support physiologiquement acceptable pour celui-ci.

32. Support particulaire comprenant un noyau solide comprenant un polysaccharide et une substance protéique, et un polymère organométallique lié au noyau destiné à être utilisé dans la fourniture de substances ayant une activité biologique à un hôte.

33. Utilisation d'un support particulaire comprenant un noyau solide comprenant un polysaccharide, une substance protéique et une substance ayant une activité biologique, et un polymère organométallique lié au noyau dans la production d'une composition immunogène pour fournir la substance ayant une activité biologique à un hôte.
